(19) Europäisches Patentamt / European Patent Office / Office européen des brevets

(11) **EP 3 643 237 B1**

(12) **EUROPEAN PATENT SPECIFICATION**

(45) Date of publication and mention of the grant of the patent:
**01.05.2024 Bulletin 2024/18**

(21) Application number: **19204837.9**

(22) Date of filing: **23.10.2019**

(51) International Patent Classification (IPC):
**A61B 5/16** *(2006.01)*    **A61B 5/374** *(2021.01)*
**A61B 5/00** *(2006.01)*    **A61B 5/378** *(2021.01)*
**G16H 50/20** *(2018.01)*

(52) Cooperative Patent Classification (CPC):
**A61B 5/165; A61B 5/374; A61B 5/378;**
**A61B 5/7264;** G16H 50/20

(54) **METHOD AND SYSTEM FOR DETERMINING CONFIDENCE LEVEL OF A PERSON USING ELECTROENCEPHALOGRAM**

VERFAHREN UND SYSTEM ZUR BESTIMMUNG DER VERTRAUENSWÜRDIGKEIT EINER PERSON UNTER VERWENDUNG EINES ELEKTROENZEPHALOGRAMMS

PROCÉDÉ ET SYSTÈME PERMETTANT DE DÉTERMINER LE NIVEAU DE CONFIANCE D'UNE PERSONNE À L'AIDE D'UN ÉLECTROENCÉPHALOGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2018 IN 201821040568**

(43) Date of publication of application:
**29.04.2020 Bulletin 2020/18**

(73) Proprietor: **Tata Consultancy Services Limited Maharashtra (IN)**

(72) Inventors:
• **GAVAS, Rahul Dasharath**
  **700160 Kolkata, West Bengal (IN)**
• **CHATTERJEE, Debatri**
  **700160 Kolkata, West Bengal (IN)**
• **SINHA, Aniruddha**
  **700160 Kolkata, West Bengal (IN)**
• **CHOWDHURY, Anirban**
  **700160 Kolkata, West Bengal (IN)**
• **SAHA, Sanjoy Kumar**
  **700032 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **MYRDEN ANDREW ET AL: "A Passive EEG-BCI for Single-Trial Detection of Changes in Mental State", IEEE TRANSACTIONS ON NEURAL SYSTEMS AND REHABILITATIONENGINEERING, IEEE SERVICE CENTER, NEW YORK, NY, US, vol. 25, no. 4, 1 April 2017 (2017-04-01), pages 345-356, XP011645704, ISSN: 1534-4320, DOI: 10.1109/TNSRE.2016.2641956 [retrieved on 2017-04-11]**
• **GAVAS RAHUL D ET AL: "Cognitive load and metacognitive confidence extraction from pupillary response", COGNITIVE SYSTEMS RESEARCH, ELSEVIER, AMSTERDAM, NL, vol. 52, 23 July 2018 (2018-07-23), pages 325-334, XP085537802, ISSN: 1389-0417, DOI: 10.1016/J.COGSYS.2018.07.021**

**Description**

Cross-Reference To Related Applications And Priority

The present application claims priority to Indian patent application No. 201821040568, filed in India on October 26, 2018.

Technical Field

The embodiments herein generally relates to the field of confidence level measurement. More particularly, but not specifically, the invention provides a system and method for determining confidence level of a person using electroencephalogram (EEG) of the person.

Background

Majority of the day-to-day decisions are associated with a sense of confidence. Even in the absence of explicit feedback, we possess an awareness of the goodness of the decisions made. Assessment of confidence is crucial as it is a major indicator of cognitive impairments like obsessive-compulsive disorder (OCD) and anxiety. A "checking" behavior is seen in subjects with reduced confidence level in their own memory. Thus confidence or the capability of being aware of the goodness of the self-performance is vital for guiding adaptive behavior in cases which lack direct feedback from the surroundings.

Metacognitive confidence is defined as the confidence generated from the observation and critical analysis of one's own the decision making process. The metacognitive confidence is very significant when in assessing the accuracy of our thinking and decision making process, while any external feedback from the environment is not present.

The metacognitive confidence governs the process of our response to a situation by pondering on whether to act immediately, or to wait and gather further evidence before deciding to act, or modify the existing world model with newly found evidences. Indeed, the higher is the level of uncertainty in the external events in the environment, the higher is the need to be attentive in information gathering to boost the learning process.

The evaluation or determination of confidence level of the person is an important from various points of view. Various studies have been published in the art to indicate the importance of the metacognitive confidence. Various methods have been used also to determine the confidence level of the person based on visual stimulation. Though there are some limitations regarding the metacognitive confidence estimation that need to be overcome. The pupillometry may get affected by the changes in the visual stimulation. An example of use of pupillometry can be found in GAVAS RAHUL D ET AL: "Cognitive load and metacognitive confidence extraction from pupillary response", COGNITIVE SYSTEMS RESEARCH, ELSEVIER, AMSTERDAM, NL, vol. 52, 23 July 2018 (2018-07-23). The confidence makers based upon the neural activity, developed so far depends on the invasive recordings of the single neuron activity which is not feasible to build a practical instrument for confidence measurement, which could be used in a real world scenario. In MYRDEN ANDREW ET AL: "A Passive EEG-BCI for Single-Trial Detection of Changes in Mental State", IEEE TRANSACTIONS ON NEURAL SYSTEMS AND REHABILITATIONENGINEERING, IEEE SERVICE CENTER, NEW YORK, NY, US, vol. 25, no. 4, 1 April 2017 (2017-04-01), electroencephalography is used to monitor changes in mental states.

SUMMARY

The invention is defined in the appended claims. The following presents a simplified summary of some embodiments of the disclosure in order to provide a basic understanding of the embodiments. This summary is not an extensive overview of the embodiments. It is not intended to identify key/critical elements of the embodiments or to delineate the scope of the embodiments. Its sole purpose is to present some embodiments in a simplified form as a prelude to the more detailed description that is presented below.

In view of the foregoing, an embodiment herein provides a system for determining confidence level of a person using electroencephalogram (EEG). The system comprises a display screen, an EEG sensor, a memory and a processor. The display screen present in front of the person to provide a stimulus. The EEG sensor attached on the person configured to capture electroencephalogram (EEG) signal of the person in response to the stimulus, wherein the EEG sensor is using a predefined number of electrodes for sensing. The processor further comprises a filtering module, an independent component analysis module, a reconstruction module, a decomposition module, a band power calculation module, a band power feature vector generation module, an outlier removal module, a cluster determination module, a normalized distance calculation module, a relevant feature identification module and a confidence metric generation module. The filtering module filters the captured EEG signal using a band pass filter. The independent component analysis module performs an independent component analysis (ICA) on the filtered EEG signal to remove the artifacts generated due to eye blink of the person. The reconstruction module reconstructs the filtered EEG signal after removing the artifacts. The decomposing module decomposes the reconstructed EEG signal into three frequency bands. The band power calculation module calculates band powers corresponding to each of the three frequency bands. The band power feature vector generation module generates band power feature vectors corresponding to each of a plurality of elements, wherein the plurality of elements are decided based on the predefined number of electrodes and the three frequency bands, wherein the generated band power feature vectors are represented in a feature matrix. The outlier removal module removes outliers from the feature matrix using a random sample consensus (RANSAC) method, wherein the removal results in the generation of an inliers feature matrix. The cluster determination module determines a first cluster corresponding to a high confidence condition and a second cluster corresponding to a low confidence condition for the each of the feature vectors of the inlier feature matrix. The normalized distance calculation module calculates a normalized distance between the first cluster and the second cluster for each feature vectors, wherein the normalized distance results in the generation of a normalized distance matrix. The relevant feature identification module identifies a set of relevant features with maximum separability using the normalized distance matrix based on a predefined condition involving features having statistically significant p-value difference between the low confidence and the high confidence condition. The confidence metric generation module generates a confidence metric by taking average of the set of relevant features to determine the confidence level of the person.

In another aspect the embodiment here provides a method for determining confidence level of a person using electroencephalogram (EEG). Initially, a stimulus is provided to the person using a display screen present in front of the person. In the next step, electroencephalogram (EEG) signal of the person is captured in response to the stimulus using an EEG sensor attached on the person, wherein the EEG sensor is using a predefined number of electrodes for sensing. Further, the captured EEG signal is filtered using a band pass filter. Later and an independent component analysis (ICA) is performed on the filtered EEG signal to remove the artifacts generated due to eye blink of the person. In the next step, the filtered EEG signal is reconstructed after removing the artifacts. In the next step, the reconstructed EEG signal is decomposed into three frequency bands. Band powers are then calculated corresponding to each of the three frequency bands. In the next step, band power feature vectors are generated corresponding to each of a plurality of elements, wherein the plurality of elements are decided based on the predefined number of electrodes and the three frequency bands, wherein the generated band power feature vectors are represented in a feature matrix. Further, outliers are removed from the feature matrix using a random sample consensus (RANSAC) method, wherein the removal results in the generation of an inliers feature matrix. In the next step, a first cluster corresponding to a high confidence condition and a second cluster corresponding to a low confidence condition are determined for the each of the feature vectors of the inlier feature matrix. Further, a normalized distance is calculated between the first cluster and the second cluster for each feature vectors, wherein the normalized distance results in the generation of a normalized distance matrix. In the next step, a set of relevant features are identified with maximum separability using the normalized distance matrix based on a predefined condition involving features having statistically significant p-value difference between the low confidence and the high confidence condition. And finally, a confidence metric is generated by taking average of the set of relevant features to determine the confidence level of the person.

In yet another embodiment, one or more non-transitory machine readable information storage mediums comprising one or more instructions is provided. The one or more instructions when executed by one or more hardware processors causes the one or more hardware processors to perform a method for providing a stimulus to the person using a display screen present in front of the person; capturing electroencephalogram (EEG) signal of the person in response to the stimulus using an EEG sensor attached on the person, wherein the EEG sensor is using a predefined number of electrodes for sensing; filtering the captured EEG signal using a band pass filter; performing an independent component analysis (ICA) on the filtered EEG signal to remove the artifacts generated due to eye blink of the person; reconstructing the filtered EEG signal after removing the artifacts; decomposing the reconstructed EEG signal into three frequency bands; calculating band powers corresponding to each of the three frequency bands; generating band power feature vectors corresponding to each of a plurality of elements, wherein the plurality of elements are decided based on the predefined number of electrodes and the three frequency bands, wherein the generated band power feature vectors are represented in a feature matrix; removing outliers from the feature matrix using a random sample consensus (RANSAC) method, wherein the removal results in the generation of an inliers feature matrix; determining a first cluster corresponding to a high confidence condition and a second cluster corresponding to a low confidence condition for the each of the feature vectors of the inlier feature matrix; calculating a normalized distance between the first cluster and the second cluster for each feature vectors, wherein the normalized distance results in the generation of a normalized distance matrix; identifying a set of relevant features with maximum separability using the normalized distance matrix based on a predefined condition involving features having statistically significant p-value difference between the low confidence and the high confidence condition; and generating a confidence metric by taking average of the set of relevant features to determine the confidence level of the person.

It should be appreciated by those skilled in the art that any block diagram herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and so executed by a computing device or processor, whether or not such computing device or processor is explicitly shown.

## BRIEF DESCRIPTION OF THE DRAWINGS

The accompanying drawings, which are incorporated in and constitute a part of this disclosure, illustrate exemplary embodiments and, together with the description, serve to explain the disclosed principles.

Fig. 1 illustrates a block diagram of a system for determining confidence level of a person using electroencephalogram according to an embodiment of the present disclosure;

Fig. 2 shows an experimental setup of the system for determining confidence level of the person using electroencephalogram according to an embodiment of the disclosure;

Fig. 3A-3C is a flowchart illustrating the steps involved in determining confidence level of a person using electroencephalogram according to an embodiment of the present disclosure;

Fig. 4 shows a schema of an addition task stimulus according to an embodiment of the disclosure;

Fig. 5 shows a schema of an anagram task according to an embodiment of the disclosure;

## DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts.

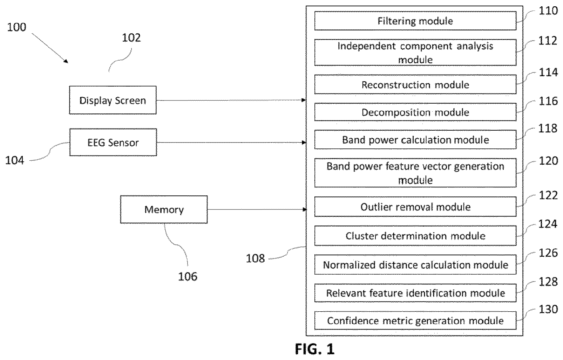

Referring now to the drawings, and more particularly to Fig. 1 through Fig. 8, where similar reference characters denote corresponding features consistently throughout the figures, there are shown preferred embodiments and these embodiments are described in the context of the following exemplary system and/or method.

According to an embodiment of the disclosure, a system 100 for determining confidence level of a person using electroencephalogram (EEG) of the person is shown in the block diagram of Fig. 1. The system 100 is configured to determine the confidence level of the person while the person is performing a task. The system 100 is used to estimate a metacognitive confidence metric based on the EEG signal, which can measure the brain activity in a non-invasive way. The confidence metric was assigned to the individual trials of two different cognitive task (Anagram task and Number Addition task which are considered as high confidence (HC) and low confidence (LC) respectively) independent of the cognitive load (CL) condition, be it high load or low load). The system and method computes a single value (scalar) measurement of the metacognitive confidence associated with the cognitive task based on a particular combination of the band power measurements of EEG acquired from different channels.

According to an embodiment of the disclosure, the system 100 further comprises a display screen 102 or an input / output module 102, an electroencephalogram sensor 104, a memory 106 and a processor 108 as shown in the block diagram of Fig. 1. The processor 108 works in communication with the memory 106. The processor 108 further comprises a plurality of modules. The plurality of modules accesses the set of algorithms stored in the memory 106 to perform a certain functions. The processor 108 further comprises a filtering module 110, an independent component analysis module 112, a reconstruction module 114, a decomposition module 116, a band power calculation module 118, a band power feature vector generation module 120, an outlier removal module 122, a cluster determination module 124, a normalized distance calculation module 126, a relevant feature identification module 128 and a confidence metric generation module 130.

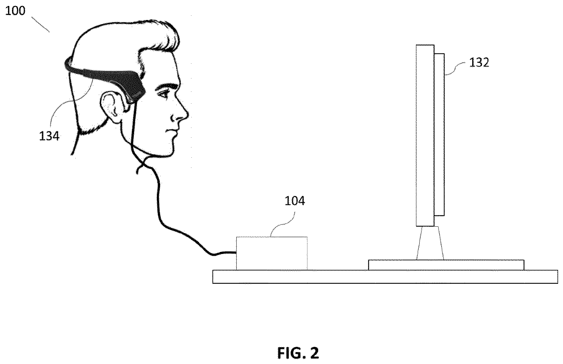

According to an embodiment of the disclosure the input/output module 102 or a display screen 102 is configured to provide a stimulus to the persons. The input/output module 102 is configured to provide one or more task to the person. In an example of the disclosure, the input/output module 102 is a computer screen 132 as shown in the experimental setup of Fig. 2. In the present example, the input/output module 102 is configured to provide an addition task and an anagram task to the person for determining the confidence level of the person. The input/output module 102 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite.

According to an embodiment of the disclosure, the system 100 is using a low cost we have used a low cost EEG headband from company Muse as the EEG sensor 104. The use of any other similar EEG sensor is well within the scope of this disclosure. The EEG sensor 104 has four dry electrodes corresponding to Fp1, Fp2, TP9 and TP10 with sampling frequency of 220 Hz. The reference electrode is at Fpz and DRLs are at a distance of one inch from the reference on both sides. The headband is worn like a pair of glasses, with the frontal electrodes placed over the middle of the forehead, and the rear electrodes placed behind the ears.

According to an embodiment of the disclosure the stimulus is presented on the display screen 102 with a resolution of 1366 * 768 pixels placed at a distance of about 60 cm from the person.

According to an embodiment of the disclosure, the system 100 comprises the filtering module 110. The filtering module 110 is configured to filter the captured EEG signal using a band pass filter. In an embodiment a 4th order band pass filter has been used. The raw EEG data x are generally contaminated by different noise sources and artifacts. Therefore data pre-processing is essential before going for the analysis of the EEG signal. The data preprocessing was done by filtering the x with a band pass filter with a pass band of about 0.5 to 40 Hz.

According to an embodiment of the disclosure, the system 100 further comprises the independent component analysis (ICA) module 112. The ICA module 112 is configured to perform an independent component analysis (ICA) on the filtered EEG signal. The ICA is performed to remove the artifacts generated due to eye blink of the person. The independent components (ICs) can be obtained from equation (1):

$$u = W*x \dots\dots\dots\dots\dots (1)$$

where, u is the matrix containing the ICs. In an example, the raw EEG data x contains 4 channels and hence equation (1) decomposes x into 4 ICs. The ICs were visually inspected and the component containing the eye-blink was removed from the u, by replacing the values of that particular component with zeros.

In an example of the disclosure, the functions to perform the band pass filtering and ICA were used from EEGLAB toolbox for MATLAB. The runica function based on the infomax ICA algorithm was used to compute the un-mixing matrix W. The use of any other tool is well within the scope of this disclosure.

According to an embodiment of the disclosure, the system 100 comprises the reconstruction module 114. The reconstruction module 114 is configured to reconstruct the filtered EEG signal after removing the artifacts. After the removal of the artifact component x was reconstructed as y using equation (2)

$$y = W{-}1 * u \dots\dots\dots\dots\dots.. (2)$$

According to an embodiment of the disclosure, the system 100 also comprises the decomposition module 116 and the band power calculation module 118. The decomposition module 116 is configured to decompose the reconstructed EEG signal into three frequency bands. The three frequency bands are namely theta in the frequency range of about 4.5 to 7.5 Hz, alpha ion the frequency range of about 8 to 12.5 Hz and beta in the frequency range of about 13 to 30Hz. Further, the band power calculation module 118 is configured to calculate the band powers corresponding to each of the three frequency bands. The band powers corresponding to each of these bands were calculated taking a 500 ms long epoch before the end time-point of each trial. This means that for the anagram task this time period is 500 ms before the mouse-click to the instant of the mouse-click and for the number addition task (which had a fixed trial period of 3 s) the time period was between 2.5 s to 3 s. The band pass filtering was done using 4th order butterworth filter. Each sample in the band-pass filtered time-period was squared and averaged over the entire period to get the band-power feature corresponding to each trial. This process was continued for all 4 EEG channels (TP9, Fp1, Fp2, and TP10).

According to an embodiment of the disclosure, the system 100 further comprises the band power feature vector generation module 120. The band power feature vector generation module 120 is configured to generate a band power feature vector corresponding to a plurality of elements. The plurality of elements are decided based on the predefined number of electrodes and the three frequency bands. Thus in the present example, after obtaining the band-powers, a band-power feature vector of 12 elements (3 frequency bands per 4 EEG channels) for all the trials comprising two different tasks for all the participants are obtained.

According to the invention, the system 100 also comprises the outlier removal module 122 and the cluster determination module 124. The outlier removal module 122 is configured to remove outliers from the feature matrix using a random sample consensus (RANSAC) method, wherein the removal results in the generation of an inliers feature matrix. Further, the cluster determination module 124 is configured to determine a first cluster corresponding to the high confidence condition (HC) and a second cluster corresponding to a low confidence (LC) condition for the each of the feature vectors of the inlier feature matrix. This has been done by plotting the boxplot of the data distribution of each feature. The example of the same has been provided in the later part of the disclosure.

According to an embodiment of the disclosure, the system 100 also comprises the normalized distance calculation module 126 and the relevant feature identification module 128. The normalized distance calculation module 126 is configured to calculate a normalized distance between the first cluster and the second cluster for each feature vectors, wherein the normalized distance results in the generation of a normalized distance matrix (Xn). The normalization of the distance of a particular feature value from a particular trial is calculated using the following pseudo code:

**IF** (feature value (Fi) falls into Cluster_HC)

THEN dist_Fi=1;

**ELSE IF** (feature value (Fi) falls into Cluster_LC)

THEN dist_Fi=0;

**ELSE**

dist=min(Fi_to_ Cluster_HC, Fi_to_Cluster_LC);

**IF**(dist is closer to Cluster_HC)

THEN dist=0.5+0.5*((std_of_Cluster_HC)/dist);

**ELSE**

dist=0.5*((std_of_Cluster_LC)/dist);

**END**

**END**

Thus the normalized distance matrix is obtained corresponding to HC and LC where it is expected in ideal case that all the values of the normalized distance matrix related to HC should be > 0.5 and values related to LC should be < 0.5. After this formulation it is seen that all the feature types which have statistically significant difference between the HC and LC groups have the same polarity. This can be seen from the Table 1 for all the significant feature types, namely: Theta_TP9, Theta_Fp2, Theta_TP10, Alpha_Fp2, Beta_TP9 and Beta_TP10

TABLE 1: THE SAMPLE MEAN AND GROUP MEAN OF VARIOUS FEATURES

| Feature Seq | Feature Name | Sample Mean of HC | Group Mean of LC | Difference in Sample Mean | p-value |
|---|---|---|---|---|---|
| 1 | Theta_TP9 | 0.3595 | 0.261 | Positive | 0.0050<0.05 |
| 3 | Theta_Fp2 | 0.6811 | 0.3867 | Positive | 0.0000<0.05 |
| 4 | Theta_TP 10 | 0.3466 | 0.2604 | Positive | 0.0131<0.05 |
| 7 | Alpha_Fp2 | 0.602 | 0.404 | Positive | 0.0000<0.05 |
| 9 | Beta_TP9 | 0.4896 | 0.3846 | Positive | 0.0109<0.05 |
| 12 | Beta_TP10 | 0.5586 | 0.4654 | Positive | 0.0195<0.05 |

The relevant feature identification module 128 is configured to identify a set of relevant features with maximum separability using the normalized distance matrix based on a predefined condition involving features having statistically significant p-value difference between the low confidence and the high confidence condition. Further, the F1-score is also used to determine the quality of separation.

The confidence metric generation module 130 is configured to generating a confidence metric by taking average of the set of relevant features. Therefore it was concluded that as the group mean difference between the sample means of the HC and LC classes are all positive (meaning that they have the same polarity), they can be averaged and get a scalar measurement of Confidence Metric (CFM) which can give significant separation between HC and LC. As per this argument CFM is defined as

$$CFM = \underline{ThetaTP9 + ThetaFP2 + ThetaTP10 + AlphaFP2 + Beta\,TP9 + Beta\,TP10}.. (3)$$

6

The confidence matric is then used to determine the confidence level of the person.

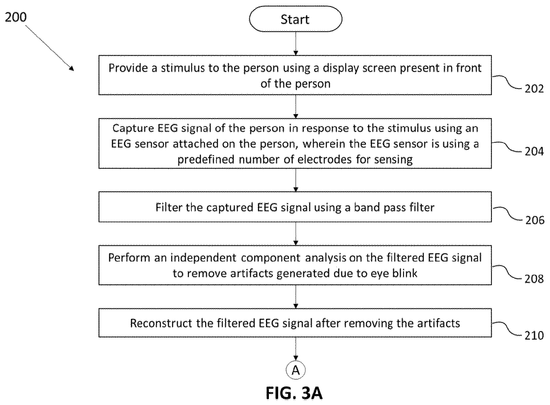

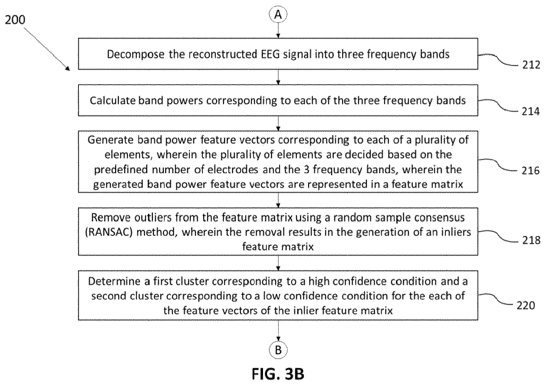

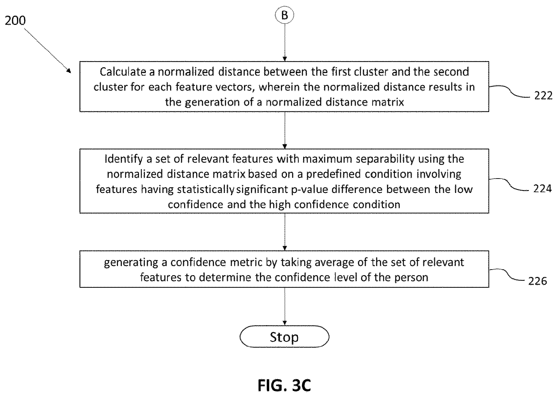

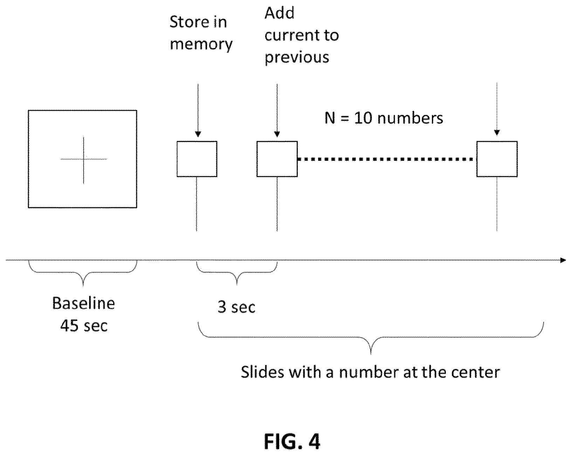

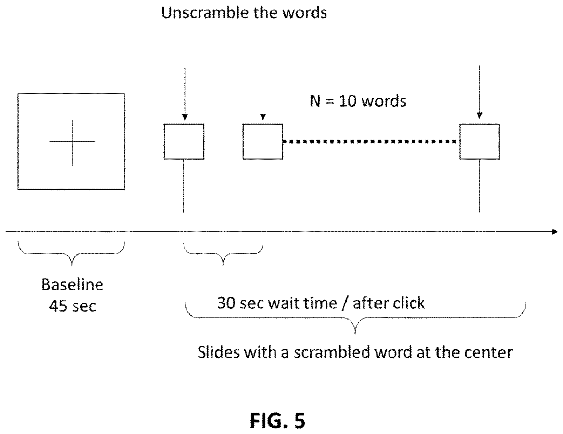

In operation, a flowchart 200 illustrating a method for determining confidence level of the person using the electroencephalogram (EEG) is shown in Fig 3A-3C. Initially at step 202, a stimulus is provided to the person using the display screen 102 present in front of the person. In an example of the disclosure, two types of stimulus are provided, the addition task and the anagram task as their schema is shown in Fig. 4 and Fig. 5 respectively. Both the tasks are designed with high cognitive load and low cognitive load. At the next step 204, the electroencephalogram (EEG) signal of the person is captured in response to the stimulus using the EEG sensor 104 attached on the person, wherein the EEG sensor 104 is using a predefined number of electrodes for sensing. In an example, the EEG headband 134 from Muse is used as the EEG sensor 104. It has four dry electrodes corresponding to Fp1, Fp2, TP9 and TP10 with sampling frequency of 220 Hz.

Further, the signal processing is performed on the captured EEG signal to remove various noise and artifacts. At step 206, the captured EEG signal is filtered using the 4th order Butterworth band pass filter. Followed by at step 208, the independent component analysis (ICA) is performed on the filtered EEG signal to remove the artifacts generated due to eye blink of the person. The independent components can be visually inspected and the component from the eye blink can be removed. In the next step 210, the filtered EEG signal is reconstructed after removing the artifacts.

In the next step, the reconstructed EEG signal is decomposed into three frequency bands namely, theta (4.5-7.5 Hz), alpha (8-12.5 Hz) and beta (13-30Hz). At step, 214, the band powers are calculated corresponding to each of the three frequency bands. The band powers are calculated taking a 500 ms long epoch before the end time-point of each trial. At step 216, the band power feature vector is generated corresponding to a plurality of elements. The plurality of elements are decided based on the predefined number of electrodes and the three frequency bands. In the present example, 4 electrodes have been used for three frequency bands, so the number of elements will be 12. The generated band power feature vectors are represented in a feature matrix;

In the next step 218, outliers are removed from the feature matrix using a random sample consensus (RANSAC) method, wherein the removal results in the generation of an inliers feature matrix. Further at step 220, a first cluster corresponding to a high confidence condition and a second cluster corresponding to a low confidence condition are determined for the each of the feature vectors of the inlier feature matrix. At step 222, the normalized distance between the first cluster and the second cluster for each feature vectors is calculated. The calculation of the normalized distance results in the generation of the normalized distance matrix (Xn).

In the next step 224, the set of relevant features with maximum separability using the normalized distance matrix are identified based on a predefined condition involving features having statistically significant p-value difference between the low confidence and the high confidence condition. The predefined condition is the normalized distance matrix values corresponding to low confidence is between 0 to 0.5 and the normalized distance matrix values corresponding to high confidence is between 0.5 to 1.0. And finally at step 226, the confidence metric is generated by taking average of the set of relevant features. The generated confidence metric is then used to determine the confidence level of the person.

According to an embodiment of the disclosure, the system 100 can also be explained with the help experimental procedures and results. In the present example, the low cost EEG headband from Muse has been used. It has four dry electrodes corresponding to Fp1, Fp2, TP9, TP10 with sampling frequency of 220 Hz. The reference electrode is at Fpz and DRLs are at a distance of one inch from the reference on both sides. The headband is worn like a pair of glasses, with the frontal electrodes placed over the middle of the forehead, and the rear electrodes placed behind the ears as shown in the experimental setup of Fig. 2. The stimulusis presented in a LCD computer screen (resolution 1366 * 768 pixels) placed at a distance of 60 cm from the participant.

Two stimulus (addition and anagram task) have been used to study the relationship between CL and the confidence level. The schema of addition task and the anagram task is shown in Fig. 4 and Fig. 5 respectively.

The following example explains how the clusters for HC and LC are determined from the boxplot. In the boxplot of feature Theta _Fp2 for HC and LC respectively. First the median of the HC and LC boxplots are compared. If median HC > median_LC then the upper-whisker of HC is used for HC_cluster and the lower-whisker of LC is used for LC_cluster, otherwise if median_LC> median_HC then the lower-whisker of HC is used for HC_cluster andthe upper-whisker of LC is used for LC_cluster. In the example, Median_LC > median_HC, therefore the lower-whisker of HC i.e. 0.0169 (25th Percentile) to 0.0003 (Lower Adjacency) is used for HC cluster and upper-whisker of LC i.e. 0.4512 (75th Percentile) to 1.0152 (Upper Adjacency) was used for LC cluster. The mean and standard deviation of the data, fallen between these lower/upper whisker ranges are taken as the cluster mean and standard deviation. Thus for the current example for feature Theta_Fp2, the cluster mean $\pm$ std for HC is 0.0101$\pm$0.0045 and the cluster mean $\pm$ std for HC is 0.6514$\pm$0.1863.

Thus the cluster mean $\pm$ std for both the classes (HC and LC) is calculated for all the 12 features (Theta_TP9, Theta Fp1,....., Beta Fp10, TP10). This is shown in Table 2.

TABLE 1: The mean and standard deviation of each cluster corresponding to each feature type

| Feature Seq | Features | Mean | Std | Mean | Std |
|---|---|---|---|---|---|
| 1 | Theta_TP9 | 1.0795 | 0.2615 | 0.0242 | 0.0135 |
| 2 | Theta Fp 1 | 0.3747 | 0.0914 | 0.021 | 0.0114 |
| 3 | Theta_Fp2 | 0.0101 | 0.0045 | 0.6514 | 0.1863 |
| 4 | Theta_TP 10 | 0.7692 | 0.1713 | 0.0287 | 0.0122 |
| 5 | Alpha_TP9 | 0.2196 | 0.0867 | 2.0138 | 0.4561 |
| 6 | Alpha_Fp1 | 0.1 | 0.0479 | 2.0123 | 0.4788 |
| 7 | Alpha_Fp2 | 0.0569 | 0.0255 | 1.5743 | 0.3363 |
| 8 | Alpha TP10 | 2.0323 | 0.5541 | 0.092 | 0.0536 |
| 9 | Beta_TP9 | 1.9915 | 0.3819 | 0.3236 | 0.2037 |
| 10 | Beta_Fp1 | 1.9124 | 0.3111 | 0.3441 | 0.1031 |
| 11 | Beta_Fp2 | 2.0272 | 0.3064 | 0.5047 | 0.1023 |
| 12 | Beta_TP10 | 0.4082 | 0.0754 | 1.7385 | 0.3475 |

Further the normalized distance matrix Xn is determined (dim: Mx12) corresponding to HC and LC where it is expected in ideal case that all the Xn values related to HC should be > 0.5 and Xn values related to LC should be < 0.5. The two-sample t-test between HC and LC based on the CFM formulation given in equation (3) yields a p-value<0.05 and the classification accuracy against the threshold 0.5 is 73.79%.

The written description describes the subject matter herein to enable any person skilled in the art to make and use the embodiments.

The embodiments of present disclosure herein solves the difficulty of detection of confidence level of the person using electroencephalogram of the person. The disclosure provides a method and system for determining confidence level using the EEG generated captured from the person in response to a stimulus.

It is to be understood that the scope of the disclosure is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

**Claims**

1. A method (200) for determining confidence level of a person using electroencephalogram (EEG), the method comprising a processor implemented steps of:

   providing a stimulus to the person using a display screen present in front of the person, wherein providing the stimulus includes providing a number addition task and an anagram task, wherein the anagram task and the number addition task are considered as high confidence (HC) and low confidence (LC) respectively;
   capturing electroencephalogram (EEG) signal of the person in response to the stimulus using an EEG sensor attached on the person (204), wherein the EEG sensor is using a predefined number of electrodes for sensing;
   filtering the captured EEG signal using a band pass filter (206);
   performing an independent component analysis (ICA) on the filtered EEG signal to remove the artifacts generated due to eye blink of the person (208);
   reconstructing the filtered EEG signal after removing the artifacts (210);
   decomposing the reconstructed EEG signal into three frequency bands, wherein the three frequency bands are theta, alpha and beta, and wherein the frequency range of theta is about 4.5 to 7.5 Hz, the frequency range of alpha is about 8 to 12.5 Hz and the frequency range of beta is about 13 to 30 Hz (212);
   calculating band powers corresponding to each of the three frequency bands (214);
   generating band power feature vectors corresponding to each of a plurality of elements, wherein the plurality of elements are decided based on the predefined number of electrodes and the three frequency bands, wherein the generated band power feature vectors are represented in a feature matrix (216);
   removing outliers from the feature matrix using a random sample consensus (RANSAC) method, wherein the removal results in the generation of an inliers feature matrix (218);
   determining a first cluster corresponding to a high confidence condition and a second cluster corresponding to a low confidence condition for the each of the feature vectors of the inlier feature matrix, wherein determining the first cluster and the second cluster by plotting a boxplot of data distribution of the each of the feature vectors for the high confidence condition and the low confidence condition comprises:

      comparing median of the high confidence condition boxplot and the low confidence condition boxplot;
      if the median of the high confidence condition boxplot is greater than the median of the low confidence condition boxplot, upper-whisker of the high confidence condition boxplot is used for the first cluster corresponding to the high confidence condition and lower-whisker of the low confidence condition boxplot is used for the second cluster corresponding to the low confidence condition, wherein the upper-whisker of the high confidence condition boxplot is ith percentile to upper adjacency and the lower-whisker of the low confidence condition boxplot is jth percentile to lower adjacency, else
      if the median of the low confidence condition boxplot is greater than the median of the high confidence condition boxplot, lower-whisker of the high confidence condition boxplot is used for the first cluster corresponding to the high confidence condition and upper-whisker of the low confidence condition boxplot is used for the second cluster corresponding to the low confidence condition, wherein the lower-whisker of the high confidence condition boxplot is ith percentile to lower adjacency and the upper-whisker of the low confidence condition boxplot is jth percentile to upper adjacency (220);

      calculating a normalized distance between the first cluster and the second cluster for each feature vectors, wherein the normalized distance results in the generation of a normalized distance matrix (222);
      identifying a set of relevant features with maximum separability using the normalized distance matrix based on a predefined condition involving features having statistically significant p-value difference between the low confidence condition and the high confidence condition (224); and
      generating a confidence metric by taking average of the set of relevant features to determine the confidence level of the person, wherein the set of relevant features averaged includes the features having group mean difference between the sample means of the high confidence condition and low confidence condition are all positive (226).

2. The method according to claim 1, wherein the predefined condition is the normalized distance matrix values corresponding to low confidence is between 0 to 0.5 and the normalized distance matrix values corresponding to high confidence is between 0.5 to 1.0.

3. The method according to claim 1, further comprising the step of calculating F1 score and the p-value from a two sample t-test to determine the quality of separation.

4. The method according to claim 1, further comprising the step of calculating the accuracy of the predicted confidence level of the person.

5. The method according to claim 1, wherein the EEG sensor is using four measuring electrodes and one neutral electrode for sensing the EEG signal of the person.

6. The method according to claim 1, wherein the band pass filter is filtering the captured EEG signal with a pass band between 0.5 Hz to 40 Hz.

7. The method of claim 1, further comprising the step of calculation of an un-mixing matrix for obtaining the independent components.

8. A system (100) for determining confidence level of a person using electroencephalogram (EEG), the system comprises:

   a display screen (102) present in front of the person to provide a stimulus, wherein providing the stimulus includes providing a number addition task and an anagram task, wherein the anagram task and the number addition task are considered as high confidence (HC) and low confidence (LC) respectively;
   an EEG sensor (104) attached on the person configured to capture electroencephalogram (EEG) signal of the person in response to the stimulus, wherein the EEG sensor is using a predefined number of electrodes for sensing;
   a memory (106); and
   a processor (108) in communication with the memory, wherein the processor further comprises:

      a filtering module (110) configured to filter the captured EEG signal using a band pass filter;
      an independent component analysis module (112) for performing an independent component analysis (ICA) on the filtered EEG signal to remove the artifacts generated due to eye blink of the person;
      a reconstruction module (114) for reconstructing the filtered EEG signal after removing the artifacts;
      a decomposing module (116) for decomposing the reconstructed EEG signal into three frequency bands, wherein the three frequency bands are theta, alpha and beta, and wherein the frequency range of theta is about 4.5 to 7.5 Hz, the frequency range of alpha is about 8 to 12.5 Hz and the frequency range of beta is about 13 to 30 Hz;
      a band power calculation module (118) for calculating band powers corresponding to each of the three frequency bands;
      a band power feature vector generation module (120) for generating band power feature vectors corresponding to each of a plurality of elements, wherein the plurality of elements are decided based on the predefined number of electrodes and the three frequency bands, wherein the generated band power feature vectors are represented in a feature matrix;
      an outlier removal module (122) for removing outliers from the feature matrix using a random sample consensus (RANSAC) method, wherein the removal results in the generation of an inliers feature matrix;
      a cluster determination module (124) for determining a first cluster corresponding to a high confidence condition and a second cluster corresponding to a low confidence condition for the each of the feature vectors of the inlier feature matrix, wherein determining the first cluster and the second cluster by plotting a boxplot of data distribution of the each of the feature vectors for the high confidence condition and the low confidence condition comprises:

         comparing median of the high confidence condition boxplot and the low confidence condition boxplot;
         if the median of the high confidence condition boxplot is greater than the median of the low confidence condition boxplot, upper-whisker of the high confidence condition boxplot is used for the first cluster corresponding to the high confidence condition and lower-whisker of the low confidence condition boxplot is used for the second cluster corresponding to the low confidence condition, wherein the upper-whisker of the high confidence condition boxplot is ith percentile to upper adjacency and the lower-whisker of the low confidence condition boxplot is jth percentile to lower adjacency, else
         if the median of the low confidence condition boxplot is greater than the median of the high confidence condition boxplot, lower-whisker of the high confidence condition boxplot is used for the first cluster corresponding to the high confidence condition and upper-whisker of the low confidence condition boxplot is used for the second cluster corresponding to the low confidence condition, wherein the lower-whisker of the high confidence condition boxplot is ith percentile to lower adjacency and the upper-whisker of the low confidence condition boxplot is jth percentile to upper adjacency ;

a normalized distance calculation module (126) for calculating an normalized distance between the first cluster and the second cluster for each feature vectors, wherein the normalized distance results in the generation of a normalized distance matrix;

a relevant feature identification module (128) for identifying a set of relevant features with maximum separability using the normalized distance matrix based on a predefined condition involving features having statistically significant p-value difference between the low confidence condition and the high confidence condition; and

a confidence metric generation module (130) for generating a confidence metric by taking average of the set of relevant features to determine the confidence level of the person, wherein the set of relevant features averaged includes the features having group mean difference between the sample means of the high confidence condition and low confidence condition are all positive .

9. A computer program product comprising a non-transitory computer readable medium having a computer readable program embodied therein, wherein the computer readable program, when executed on a computing device, causes the computing device to:

provide a stimulus to the person using a display screen present in front of the person, wherein providing the stimulus includes providing an number addition task and an anagram task, wherein the anagram task and the number addition task are considered as high confidence (HC) and low confidence (LC) respectively;

capture electroencephalogram (EEG) signal of the person in response to the stimulus using an EEG sensor attached on the person, wherein the EEG sensor is using a predefined number of electrodes for sensing;

filter the captured EEG signal using a band pass filter;

perform an independent component analysis (ICA) on the filtered EEG signal to remove the artifacts generated due to eye blink of the person;

reconstruct the filtered EEG signal after removing the artifacts;

decompose the reconstructed EEG signal into three frequency bands, wherein the three frequency bands are theta, alpha and beta, and wherein the frequency range of theta is about 4.5 to 7.5 Hz, the frequency range of alpha is about 8 to 12.5 Hz and the frequency range of beta is about 13 to 30 Hz;

calculate band powers corresponding to each of the three frequency bands;

generate band power feature vectors corresponding to each of a plurality of elements, wherein the plurality of elements are decided based on the predefined number of electrodes and the three frequency bands, wherein the generated band power feature vectors are represented in a feature matrix;

remove outliers from the feature matrix using a random sample consensus (RANSAC) method, wherein the removal results in the generation of an inliers feature matrix;

determine a first cluster corresponding to a high confidence condition and a second cluster corresponding to a low confidence condition for the each of the feature vectors of the inlier feature matrix, wherein determining the first cluster and the second cluster by plotting a boxplot of data distribution of the each of the feature vectors for the high confidence condition and the low confidence condition comprises:

comparing median of the high confidence condition boxplot and the low confidence condition boxplot;

if the median of the high confidence condition boxplot is greater than the median of the low confidence condition boxplot, upper-whisker of the high confidence condition boxplot is used for the first cluster corresponding to the high confidence condition and lower-whisker of the low confidence condition boxplot is used for the second cluster corresponding to the low confidence condition, wherein the upper-whisker of the high confidence condition boxplot is ith percentile to upper adjacency and the lower-whisker of the low confidence condition boxplot is jth percentile to lower adjacency, else

if the median of the low confidence condition boxplot is greater than the median of the high confidence condition boxplot, lower-whisker of the high confidence condition boxplot is used for the first cluster corresponding to the high confidence condition and upper-whisker of the low confidence condition boxplot is used for the second cluster corresponding to the low confidence condition, wherein the lower-whisker of the high confidence condition boxplot is ith percentile to lower adjacency and the upper-whisker of the low confidence condition boxplot is jth percentile to upper adjacency ;

calculate a normalized distance between the first cluster and the second cluster for each feature vectors, wherein the normalized distance results in the generation of a normalized distance matrix;

identify a set of relevant features with maximum separability using the normalized distance matrix based on a predefined condition involving features having statistically significant p-value difference between the low confidence condition and the high confidence condition; and

generate a confidence metric by taking average of the set of relevant features to determine the confidence level of the person, wherein the set of relevant features averaged includes the features having group mean difference between the sample means of the high confidence condition and low confidence condition are all positive .

**Patentansprüche**

1. Verfahren (200) zum Bestimmen des Konfidenzniveaus einer Person unter Verwendung von Elektroenzephalogramm (EEG), wobei das Verfahren einen Prozessor umfasst, der die folgenden Schritte implementiert:

Bereitstellen eines Reizes für die Person unter Verwendung eines Anzeigebildschirms, der vor der Person vorhanden ist, wobei das Bereitstellen des Reizes das Bereitstellen einer Nummernadditionsaufgabe und einer Anagrammaufgabe umfasst, wobei die Anagrammaufgabe und die Nummernadditionsaufgabe als hohe Konfidenz (HC) bzw. niedrige Konfidenz (LC) betrachtet werden;

Erfassen eines Elektroenzephalogramm (EEG)-Signals der Person als Reaktion auf den Reiz unter Verwendung eines EEG-Sensors, der an der Person angebracht ist (204), wobei der EEG-Sensor eine vordefinierte Anzahl von Elektroden zum Erfassen verwendet;

Filtern des erfassten EEG-Signals unter Verwendung eines Bandpassfilters (206);

Durchführen einer unabhängigen Komponentenanalyse (ICA) am gefilterten EEG-Signal, um die Artefakte zu entfernen, die aufgrund des Blinzelns der Augen der Person erzeugt werden (208);

Rekonstruieren des gefilterten EEG-Signals nach dem Entfernen der Artefakte (210);

Zerlegen des rekonstruierten EEG-Signals in drei Frequenzbänder, wobei die drei Frequenzbänder Theta, Alpha und Beta sind und wobei der Frequenzbereich von Theta etwa 4,5 bis 7,5 Hz beträgt, der Frequenzbereich von Alpha etwa 8 bis 12,5 Hz beträgt und der Frequenzbereich von Beta etwa 13 bis 30 Hz beträgt (212);

Berechnen von Bandleistungen, die jedem der drei Frequenzbänder entsprechen (214);

Erzeugen von Bandleistungsmerkmalsvektoren, die jedem einer Mehrzahl von Elementen entsprechen, wobei die Mehrzahl von Elementen basierend auf der vordefinierten Anzahl von Elektroden und den drei Frequenzbändern entschieden wird, wobei die erzeugten Bandleistungsmerkmalsvektoren in einer Merkmalsmatrix dargestellt werden (216);

Entfernen von Ausreißern aus der Merkmalsmatrix unter Verwendung eines RANSAC-Verfahrens (RANSAC: Random Sample Consensus), wobei das Entfernen zur Erzeugung einer Inliermerkmalsmatrix führt (218);

Bestimmen eines ersten Clusters, der einer hohen Konfidenzbedingung entspricht, und eines zweiten Clusters, der einer niedrigen Konfidenzbedingung entspricht, für jeden der Merkmalsvektoren der Inliermerkmalsmatrix, wobei das Bestimmen des ersten Clusters und des zweiten Clusters durch Auftragen eines Boxplots der Datenverteilung jedes der Merkmalsvektoren für die hohe Konfidenzbedingung und die niedrige Konfidenzbedingung umfasst:

Vergleichen des Medians des Boxplots der hohen Konfidenzbedingung und des Boxplots der niedrigen Konfidenzbedingung;

wenn der Median des Boxplots der hohen Konfidenzbedingung größer als der Median des Boxplots der niedrigen Konfidenzbedingung ist, wird der obere Whisker des Boxplots der hohen Konfidenzbedingung für den ersten Cluster verwendet, der der hohen Konfidenzbedingung entspricht, und der untere Whisker des Boxplots der niedrigen Konfidenzbedingung wird für den zweiten Cluster verwendet, der der niedrigen Konfidenzbedingung entspricht, wobei der obere Whisker des Boxplots der hohen Konfidenzbedingung das i-te Perzentil zur oberen Nachbarschaft ist und der untere Whisker des Boxplots der niedrigen Konfidenzbedingung das j-te Perzentil zur unteren Nachbarschaft ist, andernfalls

wenn der Median des Boxplots der niedrigen Konfidenzbedingung größer als der Median des Boxplots der hohen Konfidenzbedingung ist, wird der untere Whisker des Boxplots der hohen Konfidenzbedingung für den ersten Cluster verwendet, der der hohen Konfidenzbedingung entspricht, und der obere Whisker des Boxplots der niedrigen Konfidenzbedingung wird für den zweiten Cluster verwendet, der der niedrigen Konfidenzbedingung entspricht, wobei der untere Whisker des Boxplots der hohen Konfidenzbedingung das i-te Perzentil zur unteren Nachbarschaft ist und der obere Whisker des Boxplots der niedrigen Konfidenzbedingung das j-te Perzentil zur oberen Nachbarschaft ist (220);

Berechnen eines normalisierten Abstands zwischen dem ersten Cluster und dem zweiten Cluster für jeden Merkmalsvektor, wobei der normalisierte Abstand zur Erzeugung einer normalisierten Abstandsmatrix führt (222);

Identifizieren eines Satzes relevanter Merkmale mit maximaler Trennbarkeit unter Verwendung der normalisierten Abstandsmatrix basierend auf einer vordefinierten Bedingung, die Merkmale mit statistisch signifikanter p-Wertdifferenz zwischen der niedrigen Konfidenzbedingung und der hohen Konfidenzbedingung beinhaltet (224); und

Erzeugen einer Konfidenzmetrik durch Mittelung des Satzes relevanter Merkmale, um das Konfidenzniveau der Person zu bestimmen, wobei der gemittelte Satz relevanter Merkmale die Merkmale mit Gruppenmittelwertdifferenz zwischen den Stichprobenmitteln der hohen Konfidenzbedingung und der niedrigen Konfidenzbedingung beinhaltet, die alle positiv sind (226).

2. Verfahren nach Anspruch 1, wobei die vordefinierte Bedingung ist, dass die normalisierten Abstandsmatrixwerte, die niedriger Konfidenz entsprechen, zwischen 0 und 0,5 liegen und die normalisierten Abstandsmatrixwerte, die hoher Konfidenz entsprechen, zwischen 0,5 und 1,0 liegen.

3. Verfahren nach Anspruch 1, ferner umfassend den Schritt des Berechnens des F1-Scores und des p-Werts aus einem Zwei-Stichproben-t-Test, um die Qualität der Trennung zu bestimmen.

4. Verfahren nach Anspruch 1, ferner umfassend den Schritt des Berechnens der Genauigkeit des vorhergesagten Konfidenzniveaus der Person.

5. Verfahren nach Anspruch 1, wobei der EEG-Sensor vier Messelektroden und eine neutrale Elektrode zum Erfassen des EEG-Signals der Person verwendet.

6. Verfahren nach Anspruch 1, wobei das Bandpassfilter das erfasste EEG-Signal mit einem Durchlassband zwischen 0,5 Hz und 40 Hz filtert.

7. Verfahren nach Anspruch 1, ferner umfassend den Schritt des Berechnens einer Entmischungsmatrix zum Erhalten der unabhängigen Komponenten.

8. System (100) zum Bestimmen des Konfidenzniveaus einer Person unter Verwendung von Elektroenzephalogramm (EEG), wobei das System umfasst:

   einen Anzeigebildschirm (102), der vor der Person vorhanden ist, um einen Reiz bereitzustellen, wobei das Bereitstellen des Reizes das Bereitstellen einer Nummernadditionsaufgabe und einer Anagrammaufgabe umfasst, wobei die Anagrammaufgabe und die Nummernadditionsaufgabe als hohe Konfidenz (HC) bzw. niedrige Konfidenz (LC) betrachtet werden;

   einen EEG-Sensor (104), der an der Person angebracht ist, der konfiguriert ist, um ein Elektroenzephalogramm (EEG)-Signal der Person als Reaktion auf den Reiz zu erfassen, wobei der EEG-Sensor eine vordefinierte Anzahl von Elektroden zum Erfassen verwendet;

   einen Speicher (106); und

   einen Prozessor (108) in Kommunikation mit dem Speicher, wobei der Prozessor ferner umfasst:

      ein Filtermodul (110), das konfiguriert ist, um das erfasste EEG-Signal unter Verwendung eines Bandpassfilters zu filtern;

      ein unabhängiges Komponentenanalysemodul (112) zum Durchführen einer unabhängigen Komponentenanalyse (ICA) am gefilterten EEG-Signal, um die Artefakte zu entfernen, die aufgrund des Blinzelns der Augen der Person erzeugt werden;

      ein Rekonstruktionsmodul (114) zum Rekonstruieren des gefilterten EEG-Signals nach dem Entfernen der Artefakte;

      ein Zerlegungsmodul (116) zum Zerlegen des rekonstruierten EEG-Signals in drei Frequenzbänder, wobei die drei Frequenzbänder Theta, Alpha und Beta sind und wobei der Frequenzbereich von Theta etwa 4,5 bis 7,5 Hz beträgt, der Frequenzbereich von Alpha etwa 8 bis 12,5 Hz beträgt und der Frequenzbereich von Beta etwa 13 bis 30 Hz beträgt;

      ein Bandleistungsberechnungsmodul (118) zum Berechnen von Bandleistungen, die jedem der drei Frequenzbänder entsprechen;

      ein Bandleistungsmerkmalsvektorerzeugungsmodul (120) zum Erzeugen von Bandleistungsmerkmalsvektoren, die jedem einer Mehrzahl von Elementen entsprechen, wobei die Mehrzahl von Elementen basierend auf der vordefinierten Anzahl von Elektroden und den drei Frequenzbändern entschieden wird, wobei die erzeugten Bandleistungsmerkmalsvektoren in einer Merkmalsmatrix dargestellt werden;

ein Ausreißerentfernungsmodul (122) zum Entfernen von Ausreißern aus der Merkmalsmatrix unter Verwendung eines RANSAC-Verfahrens (RANSAC: Random Sample Consensus), wobei das Entfernen zur Erzeugung einer Inliermerkmalsmatrix führt;

ein Clusterbestimmungsmodul (124) zum Bestimmen eines ersten Clusters, der einer hohen Konfidenzbedingung entspricht, und eines zweiten Clusters, der einer niedrigen Konfidenzbedingung entspricht, für jeden der Merkmalsvektoren der Inliermerkmalsmatrix, wobei das Bestimmen des ersten Clusters und des zweiten Clusters durch Auftragen eines Boxplots der Datenverteilung jedes der Merkmalsvektoren für die hohe Konfidenzbedingung und die niedrige Konfidenzbedingung umfasst:

Vergleichen des Medians des Boxplots der hohen Konfidenzbedingung und des Boxplots der niedrigen Konfidenzbedingung;

wenn der Median des Boxplots der hohen Konfidenzbedingung größer als der Median des Boxplots der niedrigen Konfidenzbedingung ist, wird der obere Whisker des Boxplots der hohen Konfidenzbedingung für den ersten Cluster verwendet, der der hohen Konfidenzbedingung entspricht, und der untere Whisker des Boxplots der niedrigen Konfidenzbedingung wird für den zweiten Cluster verwendet, der der niedrigen Konfidenzbedingung entspricht, wobei der obere Whisker des Boxplots der hohen Konfidenzbedingung das i-te Perzentil zur oberen Nachbarschaft ist und der untere Whisker des Boxplots der niedrigen Konfidenzbedingung das j-te Perzentil zur unteren Nachbarschaft ist, andernfalls wenn der Median des Boxplots der niedrigen Konfidenzbedingung größer als der Median des Boxplots der hohen Konfidenzbedingung ist, wird der untere Whisker des Boxplots der hohen Konfidenzbedingung für den ersten Cluster verwendet, der der hohen Konfidenzbedingung entspricht, und der obere Whisker des Boxplots der niedrigen Konfidenzbedingung wird für den zweiten Cluster verwendet, der der niedrigen Konfidenzbedingung entspricht, wobei der untere Whisker des Boxplots der hohen Konfidenzbedingung das i-te Perzentil zur unteren Nachbarschaft ist und der obere Whisker des Boxplots der niedrigen Konfidenzbedingung das j-te Perzentil zur oberen Nachbarschaft ist;

ein Modul zum Berechnen eines normalisierten Abstands (126) zum Berechnen eines normalisierten Abstands zwischen dem ersten Cluster und dem zweiten Cluster für jeden Merkmalsvektor, wobei der normalisierte Abstand zur Erzeugung einer normalisierten Abstandsmatrix führt;

ein Modul zum Identifizieren relevanter Merkmale (128) zum Identifizieren eines Satzes relevanter Merkmale mit maximaler Trennbarkeit unter Verwendung der normalisierten Abstandsmatrix basierend auf einer vordefinierten Bedingung, die Merkmale mit statistisch signifikanter p-Wertdifferenz zwischen der niedrigen Konfidenzbedingung und der hohen Konfidenzbedingung beinhaltet; und

ein Konfidenzmetrikerzeugungsmodul zum Erzeugen einer Konfidenzmetrik (130) durch Mittelung des Satzes relevanter Merkmale, um das Konfidenzniveau der Person zu bestimmen, wobei der gemittelte Satz relevanter Merkmale die Merkmale mit Gruppenmittelwertdifferenz zwischen den Stichprobenmitteln der hohen Konfidenzbedingung und der niedrigen Konfidenzbedingung beinhaltet, die alle positiv sind.

9. Computerprogrammprodukt, umfassend ein nichtflüchtiges computerlesbares Medium mit einem darin enthaltenen computerlesbaren Programm, wobei das computerlesbare Programm, wenn es auf einer Rechenvorrichtung ausgeführt wird, die Rechenvorrichtung zu Folgendem veranlasst:

Bereitstellen eines Reizes für die Person unter Verwendung eines Anzeigebildschirms, der vor der Person vorhanden ist, wobei das Bereitstellen des Reizes das Bereitstellen einer Nummernadditionsaufgabe und einer Anagrammaufgabe umfasst, wobei die Anagrammaufgabe und die Nummernadditionsaufgabe als hohe Konfidenz (HC) bzw. niedrige Konfidenz (LC) betrachtet werden;

Erfassen eines Elektroenzephalogramm (EEG)-Signals der Person als Reaktion auf den Reiz unter Verwendung eines EEG-Sensors, der an der Person angebracht ist, wobei der EEG-Sensor eine vordefinierte Anzahl von Elektroden zum Erfassen verwendet;

Filtern des erfassten EEG-Signals unter Verwendung eines Bandpassfilters;

Durchführen einer unabhängigen Komponentenanalyse (ICA) am gefilterten EEG-Signal, um die Artefakte zu entfernen, die aufgrund des Blinzelns der Augen der Person erzeugt werden;

Rekonstruieren des gefilterten EEG-Signals nach dem Entfernen der Artefakte;

Zerlegen des rekonstruierten EEG-Signals in drei Frequenzbänder, wobei die drei Frequenzbänder Theta, Alpha und Beta sind und wobei der Frequenzbereich von Theta etwa 4,5 bis 7,5 Hz beträgt, der Frequenzbereich von Alpha etwa 8 bis 12,5 Hz beträgt und der Frequenzbereich von Beta etwa 13 bis 30 Hz beträgt;
Berechnen von Bandleistungen, die jedem der drei Frequenzbänder entsprechen;
Erzeugen von Bandleistungsmerkmalsvektoren, die jedem einer Mehrzahl von Elementen entsprechen, wobei die Mehrzahl von Elementen basierend auf der vordefinierten Anzahl von Elektroden und den drei Frequenzbändern entschieden wird, wobei die erzeugten Bandleistungsmerkmalsvektoren in einer Merkmalsmatrix dargestellt werden;
Entfernen von Ausreißern aus der Merkmalsmatrix unter Verwendung eines RANSAC-Verfahrens (RANSAC: Random Sample Consensus), wobei das Entfernen zur Erzeugung einer Inliermerkmalsmatrix führt;
Bestimmen eines ersten Clusters, der einer hohen Konfidenzbedingung entspricht, und eines zweiten Clusters, der einer niedrigen Konfidenzbedingung entspricht, für jeden der Merkmalsvektoren der Inliermerkmalsmatrix, wobei das Bestimmen des ersten Clusters und des zweiten Clusters durch Auftragen eines Boxplots der Datenverteilung jedes der Merkmalsvektoren für die hohe Konfidenzbedingung und die niedrige Konfidenzbedingung umfasst:

Vergleichen des Medians des Boxplots der hohen Konfidenzbedingung und des Boxplots der niedrigen Konfidenzbedingung;
wenn der Median des Boxplots der hohen Konfidenzbedingung größer als der Median des Boxplots der niedrigen Konfidenzbedingung ist, wird der obere Whisker des Boxplots der hohen Konfidenzbedingung für den ersten Cluster verwendet, der der hohen Konfidenzbedingung entspricht, und der untere Whisker des Boxplots der niedrigen Konfidenzbedingung wird für den zweiten Cluster verwendet, der der niedrigen Konfidenzbedingung entspricht, wobei der obere Whisker des Boxplots der hohen Konfidenzbedingung das i-te Perzentil zur oberen Nachbarschaft ist und der untere Whisker des Boxplots der niedrigen Konfidenzbedingung das j-te Perzentil zur unteren Nachbarschaft ist, andernfalls
wenn der Median des Boxplots der niedrigen Konfidenzbedingung größer als der Median des Boxplots der hohen Konfidenzbedingung ist, wird der untere Whisker des Boxplots der hohen Konfidenzbedingung für den ersten Cluster verwendet, der der hohen Konfidenzbedingung entspricht, und der obere Whisker des Boxplots der niedrigen Konfidenzbedingung wird für den zweiten Cluster verwendet, der der niedrigen Konfidenzbedingung entspricht, wobei der untere Whisker des Boxplots der hohen Konfidenzbedingung das i-te Perzentil zur unteren Nachbarschaft ist und der obere Whisker des Boxplots der niedrigen Konfidenzbedingung das j-te Perzentil zur oberen Nachbarschaft ist;

Berechnen eines normalisierten Abstands zwischen dem ersten Cluster und dem zweiten Cluster für jeden Merkmalsvektor, wobei der normalisierte Abstand zur Erzeugung einer normalisierten Abstandsmatrix führt;
Identifizieren eines Satzes relevanter Merkmale mit maximaler Trennbarkeit unter Verwendung der normalisierten Abstandsmatrix basierend auf einer vordefinierten Bedingung, die Merkmale mit statistisch signifikanter p-Wertdifferenz zwischen der niedrigen Konfidenzbedingung und der hohen Konfidenzbedingung beinhaltet; und
Erzeugen einer Konfidenzmetrik durch Mittelung des Satzes relevanter Merkmale, um das Konfidenzniveau der Person zu bestimmen, wobei der gemittelte Satz relevanter Merkmale die Merkmale mit Gruppenmittelwertdifferenz zwischen den Stichprobenmitteln der hohen Konfidenzbedingung und der niedrigen Konfidenzbedingung beinhaltet, die alle positiv sind.

## Revendications

1. Procédé (200) de détermination du niveau de confiance d'une personne utilisant un électroencéphalogramme (EEG), le procédé comprenant les étapes mises en oeuvre par processeur de :

fourniture d'un stimulus à la personne au moyen d'un écran d'affichage présent en face de la personne, dans lequel la fourniture du stimulus comprend la fourniture d'une tâche d'addition de nombre et d'une tâche d'anagramme, dans lequel la tâche d'anagramme et la tâche d'addition de nombre sont considérés comme étant de confiance élevée (HC) et de confiance faible (LC), respectivement ;
capture d'un signal d'électroencéphalogramme (EEG) de la personne en réponse au stimulus au moyen d'un capteur EEG fixé sur la personne (204), dans lequel le capteur EEG utilise un nombre prédéfini d'électrodes pour la détection ;
filtrage du signal EEG capturé au moyen d'un filtre passe-bande (206) ;
exécution d'une analyse en composantes indépendantes (ICA) sur le signal EEG filtré pour éliminer les artefacts générés en raison d'un clignement d'oeil de la personne (208) ;

reconstruction du signal EEG filtré après élimination des artefacts (210) ;

décomposition du signal EEG reconstruit en trois bandes de fréquence, dans lequel les trois bandes de fréquence sont thêta, alpha et bêta, et dans lequel la plage de fréquence de thêta est d'environ 4,5 à 7,5 Hz, la plage de fréquence d'alpha est d'environ 8 à 12,5 Hz et la plage de fréquence de bêta est d'environ 13 à 30 Hz (212) ;

calcul de puissances de bande correspondant à chacune des trois bandes de fréquence (214) ;

génération de vecteurs caractéristiques de puissance de bande correspondant à chacun d'une pluralité d'éléments, dans lequel la pluralité d'éléments sont décidés sur la base du nombre prédéfini d'électrodes et des trois bandes de fréquence, dans lequel les vecteurs caractéristiques de puissance de bande générés sont représentés dans une matrice de caractéristiques (216) ;

élimination des aberrants de la matrice de caractéristiques au moyen d'un procédé d'échantillon aléatoire consensus (RANSAC), dans lequel l'élimination conduit à la génération d'une matrice de caractéristiques non aberrantes (218) ;

détermination d'un premier groupe correspondant à une condition de confiance élevée et un deuxième groupe correspondant à une condition de confiance faible pour chacun des vecteurs caractéristiques de la matrice de caractéristiques non aberrantes, dans lequel la détermination du premier groupe et du deuxième groupe par tracé d'une distribution de diagramme de quartiles de données de chacun des vecteurs caractéristiques pour la condition de confiance élevée et la condition de confiance faible comprend :

la comparaison de la médiane du diagramme de quartiles de condition de confiance élevée et du diagramme de quartiles de condition de confiance faible ;

si la médiane du diagramme de quartiles de condition de confiance élevée est supérieure à la médiane du diagramme de quartiles de condition de confiance faible, le quartile supérieur du diagramme de quartiles de condition de confiance élevée est utilisé pour le premier groupe correspondant à la condition de confiance élevée et le quartile inférieur du diagramme de quartiles de condition de confiance faible est utilisé pour le deuxième groupe correspondant à la condition de confiance faible, dans lequel le quartile supérieur du diagramme de quartiles de condition de confiance élevée est le ième centile à proximité supérieure et le quartile inférieur du diagramme de quartiles de condition de confiance faible est le jème centile à proximité inférieure, sinon

si la médiane du diagramme de quartiles de condition de confiance faible est supérieure à la médiane du diagramme de quartiles de condition de confiance élevée, le quartile inférieur du diagramme de quartiles de condition de confiance élevée est utilisé pour le premier groupe correspondant à la condition de confiance élevée et le quartile supérieur du diagramme de quartiles de condition de confiance faible est utilisé pour le deuxième groupe correspondant à la condition de confiance faible, dans lequel le quartile inférieur du diagramme de quartiles de condition de confiance élevée est le ième centile à proximité inférieure et le quartile supérieur du diagramme de quartiles de condition de confiance faible est le jème centile à proximité supérieure (220) ;

calcul d'une distance normalisée entre le premier groupe et le deuxième groupe pour chaque vecteur caractéristique, dans lequel la distance normalisée conduit à la génération d'une matrice de distance normalisée (222) ;

identification d'un ensemble de caractéristiques pertinentes avec une séparabilité maximale au moyen de la matrice de distance normalisée sur la base d'une condition prédéfinie impliquant des caractéristiques ayant une différence de valeur p statistiquement significative entre la condition de confiance faible et la condition de confiance élevée (224) ; et

génération d'une métrique de confiance à partir de la moyenne de l'ensemble de caractéristiques pertinentes pour déterminer le niveau de confiance de la personne, dans lequel l'ensemble de caractéristiques pertinentes moyenné comprend les caractéristiques ayant une différence moyenne de groupe entre les moyennes d'échantillon de la condition de confiance élevée et la condition de confiance faible qui sont toutes positives (226).

**2.** Procédé selon la revendication 1, dans lequel la condition prédéfinie est que les valeurs de matrice de distance normalisée correspondant à une confiance faible sont comprises entre 0 et 0,5 et les valeurs de matrice de distance normalisée correspondant à une confiance élevée sont comprises entre 0,5 et 1,0.

**3.** Procédé selon la revendication 1, comprenant en outre l'étape de calcul de score F1 et de la valeur p à partir d'un test t à deux échantillons pour déterminer la qualité de séparation.

**4.** Procédé selon la revendication 1, comprenant en outre l'étape de calcul de la précision du niveau de confiance prédit de la personne.

**5.** Procédé selon la revendication 1, dans lequel le capteur EEG utilise quatre électrodes de mesure et une électrode neutre pour détecter le signal EEG de la personne.

**6.** Procédé selon la revendication 1, dans lequel le filtre passe-bande filtre le signal EEG capturé avec un passe-bande entre 0,5 Hz et 40 Hz.

**7.** Procédé selon la revendication 1, comprenant en outre l'étape de calcul d'une matrice de ségrégation pour obtenir les composantes indépendantes.

**8.** Système (100) pour déterminer le niveau de confiance d'une personne utilisant l'électroencéphalogramme (EEG), le système comprenant :

un écran d'affichage (102) présent en face de la personne pour fournir un stimulus, dans lequel la fourniture du stimulus comprend la fourniture d'une tâche d'addition de nombre et une tâche d'anagramme, dans lequel la tâche d'anagramme et la tâche d'addition de nombre sont considérées comme une confiance élevée (HC) et une confiance faible (LC), respectivement ;
un capteur EEG (104) fixé sur la personne configuré pour capturer un signal d'électroencéphalogramme (EEG) de la personne en réponse au stimulus, dans lequel le capteur EEG utilise un nombre prédéfini d'électrodes pour la détection ;
une mémoire (106) ; et
un processeur (108) en communication avec la mémoire, dans lequel le processeur comprend en outre :

un module de filtrage (110) configuré pour filtrer le signal EEG capturé au moyen d'un filtre passe-bande ;
un module d'analyse en composantes indépendantes (112) pour effectuer une analyse en composantes indépendantes (ICA) sur le signal EEG filtré pour éliminer les artefacts générés en raison d'un clignement d'oeil de la personne ;
un module de reconstruction (114) pour reconstruire le signal EEG filtré après élimination des artefacts ;
un module de décomposition (116) pour décomposer le signal EEG reconstruit en trois bandes de fréquence, dans lequel les trois bandes de fréquence sont thêta, alpha et bêta, et dans lequel la plage de fréquence de thêta est d'environ 4,5 à 7,5 Hz, la plage de fréquence d'alpha est d'environ 8 à 12,5 Hz et la plage de fréquence de bêta est d'environ 13 à 30 Hz ;
un module de calcul de puissance de bande (118) pour calculer des puissances de bande correspondant à chacune des trois bandes de fréquence ;
un module de génération de vecteur de caractéristique de puissance de bande (120) pour générer des vecteurs caractéristiques de puissance de bande correspondant à chacun d'une pluralité d'éléments, dans lequel la pluralité d'éléments sont décidés sur la base du nombre prédéfini d'électrodes et des trois bandes de fréquence, dans lequel les vecteurs caractéristiques de puissance de bande générés sont représentés dans une matrice de caractéristiques ;
un module d'élimination des aberrants (122) pour éliminer les aberrants de la matrice de caractéristiques au moyen d'un procédé d'échantillon aléatoire consensus (RANSAC), dans lequel l'élimination conduit à la génération d'une matrice de caractéristiques non aberrantes ;
un module de détermination de groupe (124) pour déterminer un premier groupe correspondant à une condition de confiance élevée et un deuxième groupe correspondant à une condition de confiance faible pour chacun des vecteurs caractéristiques de la matrice de caractéristiques non aberrantes, dans lequel la détermination du premier groupe et du deuxième groupe par tracé d'une distribution de diagramme de quartiles de données de chacun des vecteurs caractéristiques pour la condition de confiance élevée et la condition de confiance faible comprend :

la comparaison de la médiane du diagramme de quartiles de condition de confiance élevée et du diagramme de quartiles de condition de confiance faible ;
si la médiane du diagramme de quartiles de condition de confiance élevée est supérieure à la médiane du diagramme de quartiles de condition de confiance faible, le quartile supérieur du diagramme de quartiles de condition de confiance élevée est utilisé pour le premier groupe correspondant à la condition de confiance élevée et le quartile inférieur du diagramme de quartiles de condition de confiance faible est utilisé pour le deuxième groupe correspondant à la condition de confiance faible, dans lequel le quartile supérieur du diagramme de quartiles de condition de confiance élevée est le ième centile à proximité supérieure et le quartile inférieur du diagramme de quartiles de condition de confiance faible est le jème centile à proximité inférieure, sinon

17

si la médiane du diagramme de quartiles de condition de confiance faible est supérieure à la médiane du diagramme de quartiles de condition de confiance élevée, le quartile inférieur du diagramme de quartiles de condition de confiance élevée est utilisé pour le premier groupe correspondant à la condition de confiance élevée et le quartile supérieur du diagramme de quartiles de condition de confiance faible est utilisé pour le deuxième groupe correspondant à la condition de confiance faible, dans lequel le quartile inférieur du diagramme de quartiles de condition de confiance élevée est le ième centile à proximité inférieure et le quartile supérieur du diagramme de quartiles de condition de confiance faible est le jème centile à proximité supérieure ;

un module de calcul de distance normalisée (126) pour calculer une distance normalisée entre le premier groupe et le deuxième groupe pour chaque vecteur caractéristique, dans lequel la distance normalisée conduit à la génération d'une matrice de distance normalisée ;
un module d'identification de caractéristiques pertinentes (128) pour identifier un ensemble de caractéristiques pertinentes avec une séparabilité maximale au moyen de la matrice de distance normalisée sur la base d'une condition prédéfinie impliquant des caractéristiques ayant une différence de valeur p statistiquement significative entre la condition de confiance faible et la condition de confiance élevée ; et
un module de génération de métrique de confiance (130) pour générer une métrique de confiance à partir de la moyenne de l'ensemble de caractéristiques pertinentes pour déterminer le niveau de confiance de la personne, dans lequel l'ensemble de caractéristiques pertinentes moyenné comprend les caractéristiques ayant une différence moyenne de groupe entre les moyennes d'échantillon de la condition de confiance élevée et la condition de confiance faible qui sont toutes positives.

9. Produit de programme informatique comprenant un support lisible par ordinateur non transitoire comportant un programme lisible par ordinateur réalisé dans celui-ci, dans lequel le programme lisible par ordinateur, lorsqu'il est exécuté sur un dispositif informatique, amène le dispositif informatique à :

fournir un stimulus à la personne au moyen d'un écran d'affichage présent en face de la personne, dans lequel la fourniture du stimulus comprend la fourniture d'une tâche d'addition de nombre et d'une tâche d'anagramme, dans lequel la tâche d'anagramme et la tâche d'addition de nombre sont considérés comme étant de confiance élevée (HC) et de confiance faible (LC), respectivement ;
capturer un signal d'électroencéphalogramme (EEG) de la personne en réponse au stimulus au moyen d'un capteur EEG fixé sur la personne, dans lequel le capteur EEG utilise un nombre prédéfini d'électrodes pour la détection ;
filtrer le signal EEG capturé au moyen d'un filtre passe-bande ;
effectuer une analyse en composantes indépendantes (ICA) sur le signal EEG filtré pour éliminer les artefacts générés en raison d'un clignement d'oeil de la personne ;
reconstruire le signal EEG filtré après élimination des artefacts ;
décomposer le signal EEG reconstruit en trois bandes de fréquence, dans lequel les trois bandes de fréquence sont thêta, alpha et bêta, et dans lequel la plage de fréquence de thêta est d'environ 4,5 à 7,5 Hz, la plage de fréquence d'alpha est d'environ 8 à 12,5 Hz et la plage de fréquence de bêta est d'environ 13 à 30 Hz ;
calculer des puissances de bande correspondant à chacune des trois bandes de fréquence ;
générer des vecteurs caractéristiques de puissance de bande correspondant à chacun d'une pluralité d'éléments, dans lequel la pluralité d'éléments sont décidés sur la base du nombre prédéfini d'électrodes et des trois bandes de fréquence, dans lequel les vecteurs caractéristiques de puissance de bande générés sont représentés dans une matrice de caractéristiques ;
éliminer les aberrants de la matrice de caractéristiques au moyen d'un procédé d'échantillon aléatoire consensus (RANSAC), dans lequel l'élimination conduit à la génération d'une matrice de caractéristiques non aberrantes ;
déterminer un premier groupe correspondant à une condition de confiance élevée et un deuxième groupe correspondant à une condition de confiance faible pour chacun des vecteurs caractéristiques de la matrice de caractéristiques non aberrantes, dans lequel la détermination du premier groupe et du deuxième groupe par tracé d'une distribution de diagramme de quartiles de données de chacun des vecteurs caractéristiques pour la condition de confiance élevée et la condition de confiance faible comprend :

la comparaison de la médiane du diagramme de quartiles de condition de confiance élevée et du diagramme de quartiles de condition de confiance faible ;
si la médiane du diagramme de quartiles de condition de confiance élevée est supérieure à la médiane du diagramme de quartiles de condition de confiance faible, le quartile supérieur du diagramme de quartiles de condition de confiance élevée est utilisé pour le premier groupe correspondant à la condition de confiance élevée et le quartile inférieur du diagramme de quartiles de condition de confiance faible est utilisé pour le deuxième groupe correspondant à la condition de confiance faible, dans lequel le quartile supérieur du diagramme de quartiles de condition de confiance élevée est le ième centile à proximité supérieure et le quartile inférieur du diagramme de quartiles de condition de confiance faible est le jème centile à proximité inférieure, sinon

si la médiane du diagramme de quartiles de condition de confiance faible est supérieure à la médiane du diagramme de quartiles de condition de confiance élevée, le quartile inférieur du diagramme de quartiles de condition de confiance élevée est utilisé pour le premier groupe correspondant à la condition de confiance élevée et le quartile supérieur du diagramme de quartiles de condition de confiance faible est utilisé pour le deuxième groupe correspondant à la condition de confiance faible, dans lequel le quartile inférieur du diagramme de quartiles de condition de confiance élevée est le ième centile à proximité inférieure et le quartile supérieur du diagramme de quartiles de condition de confiance faible est le jème centile à proximité supérieure ;

calculer une distance normalisée entre le premier groupe et le deuxième groupe pour chaque vecteur caractéristique, dans lequel la distance normalisée conduit à la génération d'une matrice de distance normalisée ;
identifier un ensemble de caractéristiques pertinentes avec une séparabilité maximale au moyen de la matrice de distance normalisée sur la base d'une condition prédéfinie impliquant des caractéristiques ayant une différence de valeur p statistiquement significative entre la condition de confiance faible et la condition de confiance élevée ; et
générer une métrique de confiance à partir de la moyenne de l'ensemble de caractéristiques pertinentes pour déterminer le niveau de confiance de la personne, dans lequel l'ensemble de caractéristiques pertinentes moyenné comprend les caractéristiques ayant une différence moyenne de groupe entre les moyennes d'échantillon de la condition de confiance élevée et la condition de confiance faible qui sont toutes positives.

**FIG. 1**

**FIG. 2**

200

```
          ┌───────────────┐
          │     Start      │
          └───────┬───────┘
                  │
                  ▼
┌──────────────────────────────────────────────────┐
│ Provide a stimulus to the person using a display   │
│ screen present in front of the person              │──── 202
└──────────────────────┬───────────────────────────┘
                       │
                       ▼
┌──────────────────────────────────────────────────┐
│ Capture EEG signal of the person in response to    │
│ the stimulus using an EEG sensor attached on the   │──── 204
│ person, wherein the EEG sensor is using a          │
│ predefined number of electrodes for sensing        │
└──────────────────────┬───────────────────────────┘
                       │
                       ▼
┌──────────────────────────────────────────────────┐
│ Filter the captured EEG signal using a band pass   │──── 206
│ filter                                             │
└──────────────────────┬───────────────────────────┘
                       │
                       ▼
┌──────────────────────────────────────────────────┐
│ Perform an independent component analysis on the   │
│ filtered EEG signal to remove artifacts generated  │──── 208
│ due to eye blink                                   │
└──────────────────────┬───────────────────────────┘
                       │
                       ▼
┌──────────────────────────────────────────────────┐
│ Reconstruct the filtered EEG signal after removing │──── 210
│ the artifacts                                      │
└──────────────────────┬───────────────────────────┘
                       │
                       ▼
                      (A)
```

**FIG. 3A**

22

200

(A)

| Decompose the reconstructed EEG signal into three frequency bands | 212 |

| Calculate band powers corresponding to each of the three frequency bands | 214 |

| Generate band power feature vectors corresponding to each of a plurality of elements, wherein the plurality of elements are decided based on the predefined number of electrodes and the 3 frequency bands, wherein the generated band power feature vectors are represented in a feature matrix | 216 |

| Remove outliers from the feature matrix using a random sample consensus (RANSAC) method, wherein the removal results in the generation of an inliers feature matrix | 218 |

| Determine a first cluster corresponding to a high confidence condition and a second cluster corresponding to a low confidence condition for the each of the feature vectors of the inlier feature matrix | 220 |

(B)

**FIG. 3B**

200

(B)

Calculate a normalized distance between the first cluster and the second cluster for each feature vectors, wherein the normalized distance results in the generation of a normalized distance matrix — 222

Identify a set of relevant features with maximum separability using the normalized distance matrix based on a predefined condition involving features having statistically significant p-value difference between the low confidence and the high confidence condition — 224

generating a confidence metric by taking average of the set of relevant features to determine the confidence level of the person — 226

Stop

**FIG. 3C**

Store in
memory

Add
current to
previous

N = 10 numbers

Baseline
45 sec

3 sec

Slides with a number at the center

**FIG. 4**

Unscramble the words

N = 10 words

Baseline
45 sec

30 sec wait time / after click

Slides with a scrambled word at the center

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201821040568

**Non-patent literature cited in the description**

- Cognitive load and metacognitive confidence extraction from pupillary response. **GAVAS RAHUL D et al.** COGNITIVE SYSTEMS RESEARCH. ELSEVIER, 23 July 2018, vol. 52

- A Passive EEG-BCI for Single-Trial Detection of Changes in Mental State. **MYRDEN ANDREW et al.** IEEE TRANSACTIONS ON NEURAL SYSTEMS AND REHABILITATIONENGINEERING. IEEE SERVICE CENTER, 01 April 2017, vol. 25